# FASCICULE DE BREVET EUROPEEN

(11) **EP 3 008 452 B1**
(45) Date de publication et mention de la délivrance du brevet: **17.08.2022**
(21) Numéro de dépôt: 14728992.0
(22) Date de dépôt: 10.06.2014
(51) Int. Cl.: G01N 21/51, G01N 21/64, G01N 21/01, G01N 21/47

(54) **SYSTÈME ET PROCÉDÉ DE DÉTECTION PERMETTANT DE DÉTECTER LA PRÉSENCE D'UN MICRO-ORGANISME DANS UN ÉCHANTILLON À L'INTÉRIEUR D'UN CONTENANT**
NACHWEISSYSTEM UND VERFAHREN ZUM NACHWEIS DER ANWESENHEIT EINES MIKROORGANISMUS IN EINER PROBE IN EINEM BEHÄLTER
DETECTION SYSTEM AND METHOD MAKING IT POSSIBLE TO DETECT THE PRESENCE OF A MICRO-ORGANISM IN A SAMPLE INSIDE A CONTAINER

(30) Priorité: 11.06.2013 FR 1355394
(43) Date de publication de la demande: 20.04.2016
(73) Titulaire: Biomérieux, 69280 Marcy l'Étoile (FR)
(72) Inventeur: COLIN, Bruno, F-69280 Marcy l'Étoile (FR); DE LA FOATA, Corinne, Chicago, Illinois 60630 (US); DACHAUD, Jacques, F-25000 Besançon (FR); MADE, Fabienne, F-69130 Ecully (FR)
(74) Mandataire: bioMérieux PI Groupement mandataires
(86) Numéro de dépôt international: PCT/EP2014/062024
(87) Numéro de publication internationale: WO 2014/198718

(56) Documents cités:
- EP-A2- 0 635 570
- WO-A1-94/07123
- WO-A2-2012/016159
- US-A- 5 432 061
- US-A- 5 516 692
- US-A- 5 770 394
- US-A1- 2005 254 055

## Description

### Domaine Technique

L'invention concerne un système de détection comprenant un dispositif de détection et un procédé de détection permettant de détecter la présence d'un micro-organisme dans un échantillon localisé à l'intérieur d'un contenant, ledit échantillon étant en contact avec un milieu de culture, et susceptible de contenir un micro-organisme. La présence d'un microorganisme est déterminée en fonction de l'apparition d'une éventuelle croissance microbienne à l'intérieur du contenant. La présence d'une telle croissance microbienne peut se traduire, par exemple, par l'apparition de troubles au sein du milieu de culture.

### Etat de la technique

La possibilité d'observer l'apparition d'une éventuelle croissance microbienne à l'intérieur d'un contenant peut s'avérer utile pour une large variété de procédures industrielles et biologiques, notamment concernant les tests de stérilité pour des procédures utilisées lors de la production de produits pharmaceutiques.

Le « Media-Fill-Test » (MFT) est un exemple de test selon l'art antérieur concernant les procédés d'observation de l'apparition d'une éventuelle croissance microbienne. Ce test, réalisé au sein de l'industrie pharmaceutique, permet de vérifier si des procédés permettant de fabriquer des produits stériles sont exécutés sans aucune menace de contamination microbiologique. Ainsi, lorsqu'une contamination microbiologique du produit supposé être stérile se produit lors de l'exécution d'un procédé permettant d'obtenir le produit stérile, cette contamination est détectée ultérieurement lors de la mise en culture du produit supposé être stérile.

La technique du MFT permet également de vérifier, lors du contrôle d'une éventuelle contamination microbiologique, si les protocoles suivis par le personnel sont respectés et surtout efficaces pour éviter de potentielles contaminations microbiennes.

Une des particularités du MFT consiste en ce que les procédés, permettant la fabrication de produits stériles, imposent la forme et la taille du contenant dont le contenu comprend l'échantillon et le milieu de culture. Ainsi, le contenant, dans lequel une éventuelle apparition d'une croissance microbienne peut être observée, peut se présenter sous différentes dimensions et formes. Pour cette raison, l'automatisation, à l'aide du MFT, des procédures de suivi d'une éventuelle croissance microbienne dans les contenants, nécessite une adaptation de chaque procédure de test à la dimension et à la forme du contenant concerné, occasionnant une perte de temps non négligeable. Dans l'art antérieur, le contrôle des produits soumis à des procédures et protocoles visant à s'assurer de la parfaite stérilité desdits produits est réalisé par un technicien qui observe le contenant de façon ponctuelle dans le but de détecter l'apparition d'une croissance microbienne. L'apparition d'une croissance microbienne peut se manifester par la présence d'une turbidité à l'intérieur desdits contenants. La turbidité constitue un exemple d'un critère ou d'un paramètre d'analyse représentant la croissance microbienne. Un autre critère souvent utilisé est l'apparition ou la disparition de la fluorescence d'un fluide. Lorsque le critère de croissance microbienne utilisé est l'observation de la disparition de la fluorescence, le technicien doit régulièrement, de manière ponctuelle, noter ses observations et effectuer des opérations visant à contrôler l'éventuelle présence de ladite fluorescence à l'intérieur du contenant. La mobilisation du technicien représente une contrainte lorsque plusieurs opérations de contrôle relatives à différents contenants doivent être réalisées.

Les acteurs de l'industrie pharmaceutique ont récemment reconnu qu'il serait fort utile de pouvoir suivre l'évolution de l'éventuelle croissance microbienne, de manière continue. Ce suivi pourrait être effectué en assurant la traçabilité informatique des résultats et la gestion informatique de l'apparition de troubles pour une quantité importante de tests. Par ailleurs, la réduction voire la suppression de la manipulation des contenants s'avère nécessaire afin de faciliter les procédures de test.

De plus, l'observation actuelle de cette éventuelle croissance microbienne est basée sur un critère ou un paramètre d'analyse unique pour une même observation. Il s'avère nécessaire de permettre la détection d'une croissance microbienne basée sur au moins deux critères ou deux paramètres d'analyse au cours d'une même observation afin d'améliorer la fiabilité de l'observation de la croissance microbienne. Des dispositifs et systèmes déjà connus de l'art antérieur permettent de suivre une éventuelle croissance microbienne à l'intérieur d'un récipient.

Un premier type de système connu de l'art antérieur est divulgué au sein de la demande de brevet américain US 2005/0266516. Ce système comprend un contenant d'analyse destiné à recevoir un fluide afin de détecter la présence ou l'absence de la fluorescence ou encore l'observation de l'évolution de la turbidité dudit fluide au cours du temps. Le fonctionnement du système selon le document US 2005/0266516 est basé sur l'association du contenant d'analyse avec un support de contenant spécifiquement adapté pour recevoir le contenant d'analyse. Ainsi, lorsqu'un fluide à analyser est contenu dans un premier contenant, le fluide doit être transféré dans un deuxième contenant spécifique tel que le contenant d'analyse afin que l'analyse puisse être réalisée, à l'aide du support de contenant, pour observer une éventuelle croissance microbienne. Le support de contenant comprend en son intérieur des sources lumineuses et des moyens de détection afin de détecter la présence de fluorescence, l'absence de fluorescence ou la présence de turbidité au sein du contenant d'analyse. Par conséquent, le système selon le document US 2005/0266516 nécessite d'utiliser un contenant spécifique tel que le contenant d'analyse préformé de manière à s'adapter à la forme et à la dimension du support de contenant. Un tel système impose donc un transfert du fluide à analyse d'un premier contenant vers un deuxième contenant. L'utilisation d'un tel système lors d'un MFT n'est pas adaptée. En effet, le transfert d'un fluide d'un premier contenant vers un deuxième contenant est extrêmement préjudiciable à la stérilité du fluide, le risque de contamination microbiologique lors du transfert de fluide étant élevé.

De manière similaire, un deuxième type de système connu de l'art antérieur est divulgué au sein de la demande de brevet US 6,723,554. Ce deuxième système comprend un contenant d'analyse destiné à recevoir un fluide afin de détecter la présence ou l'absence de la fluorescence ou encore l'observation de l'évolution de la turbidité dudit fluide au cours du temps. Le fonctionnement du système selon le document US 6,723,554 est basé sur l'association du contenant d'analyse avec un support de contenant spécifiquement adapté pour recevoir le contenant d'analyse. Ainsi, lorsqu'un fluide à analyser est contenu dans un premier contenant, le fluide doit être transféré dans un deuxième contenant spécifique tel que le contenant d'analyse afin que l'analyse puisse être réalisée pour observer une éventuelle croissance microbienne. Le support de contenant est préformé afin de permettre l'insertion d'une source lumineuse et d'un moyen de détection depuis l'extérieur de la paroi du support de contenant jusqu'à la paroi du contenant. Par conséquent, le système selon le document US 6,723,554 nécessite d'utiliser un contenant spécifique tel que le contenant d'analyse avec une forme et une dimension spécifiquement adaptée à la forme et à la dimension du support de contenant. Un tel système impose donc également un transfert du fluide à analyser d'un premier contenant vers un deuxième contenant spécifique tel que le contenant d'analyse. De manière similaire au premier type de système de l'art antérieur, le deuxième type de système de l'art antérieur nécessite un transfert du fluide à analyser d'un premier contenant vers un deuxième contenant possédant une forme et une dimension adaptées pour correspondre à la forme et à la dimension d'un support de contenant nécessaire à l'analyse du fluide.

Ainsi, de tels systèmes entraînent une augmentation du risque de contamination lors du procédé d'analyse du fluide. Il s'avère donc nécessaire de permettre l'analyse d'un fluide en utilisant un seul et même contenant.

De plus, de tels systèmes ne sont adaptés que pour un type de contenant spécifique. Ainsi, lorsqu'un grand nombre d'analyse doit être réalisé, les coûts d'analyse relatifs à la production de contenants identiques sont importants. Il s'avère donc également nécessaire de permettre l'analyse d'un fluide au sein d'un contenant, quelle que soit la forme et/ou la dimension du contenant afin de limiter les coûts relatifs à la mise en oeuvre du procédé d'analyse du fluide.

Le document US2005254055-A1 divulgue un système de surveillance de culture comprenant un appareil et un procédé de surveillance en temps réel et en ligne d'une culture cellulaire biologique dans un conteneur. Le système comprend un récipient pour contenir un milieu de culture biologique liquide, des sources d'émission de lumière émettant de la lumière pour interagir avec le milieu de culture biologique à travers une paroi transparente d'un récipient, des photodétecteurs détectant directement la lumière diffusée ou transmise, une fixation de sonde pour contenir les sources et des photodétecteurs l'un par rapport à l'autre et pour maintenir le récipient, et un processeur.

### Objet de l'invention

En référence aux observations ci-dessus, un objectif de la présente invention consiste à fournir un système de détection comprenant un dispositif de détection et un procédé de détection pour détecter la présence d'un micro-organisme dans un échantillon localisé à l'intérieur d'un contenant, ledit échantillon étant en contact avec un milieu de culture, et susceptible de contenir un micro-organisme, tout en évitant les problèmes et inconvénients liés aux dispositifs, procédés et systèmes relatifs à l'art antérieur.

Un autre objectif consiste à fournir un système de détection comprenant un dispositif de détection et un procédé de détection grâce auxquels une éventuelle présence microbienne peut être détectée à l'intérieur d'une variété de contenants, quelle que soit la forme et la dimension de ces contenants. Un autre objectif consiste à fournir un dispositif, un système et un procédé de détection permettant la détection d'une présence microbienne à l'intérieur d'un contenant sans interrompre le déroulement du procédé. Résumé de l'invention Ainsi, selon un premier aspect de l'invention, la présente invention concerne un système de détection comprenant un dispositif de détection de la présence d'au moins un micro-organisme dans le contenu d'un contenant comprenant une paroi avec une zone translucide, ledit dispositif de détection comprenant : a) au moins une source lumineuse, telle qu'une diode électroluminescente (LED), susceptible d'illuminer le contenu du contenant en émettant un faisceau lumineux d'excitation au travers de la zone translucide du contenant ; b) au moins un moyen de détection, tel qu'une photodiode, pour détecter au moins un faisceau lumineux de réaction émis en réponse à l'illumination du contenu du contenant ; ladite au moins une source lumineuse et ledit au moins un moyen de détection étant pourvus d'au moins un moyen de connexion entièrement localisé à l'extérieur du contenant pour connecter ladite au moins une source lumineuse et ledit au moins un moyen de détection à la paroi du contenant, au niveau de la zone translucide, ledit moyen de connexion permettant d'adapter la position du dispositif de détection sur la paroi du contenant, ledit au moins un moyen de détection étant positionné selon un angle de valeur déterminée par rapport à la direction du faisceau lumineux d'excitation pour détecter le faisceau lumineux de réaction.

De manière avantageuse, le moyen de détection comprend au moins un premier et un deuxième photodétecteur, respectivement positionnés à un premier et à un deuxième emplacement pour détecter, au niveau de la zone translucide du contenant, un premier et un deuxième faisceau lumineux de réaction et ce, afin d'obtenir une première et une deuxième valeur d'un paramètre d'analyse représentatif d'une éventuelle présence microbienne dans le contenu du contenant.

De manière avantageuse, ledit au moins un moyen de détection comprend un premier photodétecteur adapté pour détecter un premier signal lumineux de réaction afin d'obtenir une valeur n d'un premier paramètre d'analyse représentatif d'une éventuelle présence microbienne dans le contenu du contenant et un deuxième photodétecteur, adapté pour détecter un deuxième faisceau lumineux de réaction, différent du premier faisceau lumineux de réaction, afin d'obtenir une valeur m d'un deuxième paramètre d'analyse représentatif d'une éventuelle présence microbienne dans le contenu du contenant.

De manière avantageuse, le premier photodétecteur comprend une photodiode filtrée dans le rouge et le deuxième photodétecteur comprend une photodiode filtrée dans le vert.

Le système de détection selon la présente invention comprend un dispositif de détection et un dispositif de contrôle connecté à ladite au moins une source lumineuse et audit au moins un moyen de détection pour contrôler le faisceau lumineux d'excitation émis par ladite au moins une source lumineuse et traiter et/ou analyser ledit au moins un faisceau lumineux de réaction émis en réponse à l'illumination du contenu du contenant, et détecté par ledit au moins un moyen de détection. De manière avantageuse, le dispositif de contrôle comprend :
- un moyen de stockage pour stocker une première valeur du premier et du deuxième paramètre d'analyse obtenue avec au moins un moyen de détection et une deuxième valeur du premier et du deuxième paramètre d'analyse obtenue après une durée de temps déterminée avec au moins un moyen de détection, - un moyen de comparaison pour comparer la première valeur et la deuxième valeur du premier et du deuxième paramètre d'analyse pour déterminer une éventuelle croissance microbienne dans le contenu du contenant. De manière avantageuse, le dispositif de contrôle est adapté pour recevoir et stocker des valeurs obtenues avec ledit au moins un moyen de détection, de manière continue.

De manière avantageuse, le dispositif de contrôle comprend une alarme, pour indiquer une éventuelle présence microbienne dans le contenu du contenant.

Selon un deuxième aspect de l'invention, la présente invention concerne un procédé de détection de la présence d'au moins un micro-organisme dans un échantillon susceptible de contenir ledit au moins un micro-organisme, ledit procédé comprenant les étapes suivantes : a) introduire l'échantillon dans un contenant comprenant une paroi avec au moins une zone translucide, ledit échantillon étant mis en contact avec un milieu de culture préalablement à l'introduction ou après l'introduction dudit échantillon au sein dudit contenant, ledit au moins un milieu de culture étant adapté pour permettre la croissance dudit au moins un micro-organisme, le mélange de l'échantillon et dudit milieu de culture formant tout ou partie du contenu du contenant, b) éventuellement incuber le contenant à une température et durant un laps de temps suffisants pour permettre la croissance dudit au moins un micro-organisme, c) mesurer, à l'aide d'un système de détection selon la présente invention au moins une valeur n d'au moins un paramètre d'analyse représentatif d'une éventuelle présence microbienne à l'intérieur du contenant, l'étape c) comprenant les sous-étapes consistant à : cl) illuminer le contenu du contenant au travers de la zone translucide avec la source lumineuse et, c2) détecter le faisceau lumineux de réaction émis en réponse à l'illumination du contenu avec ledit au moins un moyen de détection afin d'obtenir ladite valeur n du paramètre d'analyse, d) comparer ladite valeur n avec une valeur seuil ns du même paramètre d'analyse, ladite valeur seuil ns étant indicative de la présence du au moins un microorganisme dans l'échantillon, e) déduire la présence ou l'absence dudit au moins un micro-organisme, au sein de l'échantillon, à partir du résultat de la comparaison.

De préférence, l'étape b) consiste à incuber le contenant à une température et durant un laps de temps suffisants pour permettre la croissance dudit au moins un micro-organisme. Selon un mode réalisation particulier du procédé selon la présente invention,
- l'étape c) comprend mesurer au moins un paramètre d'analyse dont la valeur n croît lorsque la quantité dudit micro-organisme croît, comme par exemple la turbidité, - l'étape d) comprend comparer ladite valeur n avec une valeur seuil ns du même paramètre d'analyse, ladite valeur seuil ns étant indicative de la présence du au moins un micro-organisme dans l'échantillon,
- l'étape e) comprend déduire la contamination de l'échantillon par ledit au moins un micro-organisme lorsque la valeur n est supérieure ou égale à la valeur seuil ns. Selon une variante du mode de réalisation particulier du procédé selon la présente invention :
- l'étape c) comprend mesurer au moins un paramètre d'analyse dont la valeur n décroît lorsque la quantité dudit micro-organisme décroît, comme par exemple la fluorescence,
- l'étape d) comprend comparer ladite valeur n avec une valeur seuil ns du même paramètre d'analyse,
- l'étape e) comprend déduire la contamination de l'échantillon par ledit au moins un micro-organisme lorsque la valeur n est inférieure ou égale à la valeur seuil ns.

Selon une variante du mode de réalisation particulier du procédé selon la présente invention:
- l'étape c) comprend mesurer au moins un premier paramètre d'analyse dont une valeur ni croît lorsque la quantité dudit micro-organisme croît, comme par exemple la turbidité et mesurer au moins un deuxième paramètre d'analyse dont une valeur n2 décroît lorsque la quantité dudit micro-organisme décroît, comme par exemple la fluorescence,
- l'étape d) comprend comparer ladite valeur ni avec une valeur seuil nsl associée au premier paramètre d'analyse et comparer ladite valeur n2 avec une valeur seuil ns2 associée au deuxième paramètre d'analyse, - l'étape e) comprend la déduction de la contamination de l'échantillon par ledit au moins un micro-organisme lorsque la valeur ni est supérieure ou égale à la valeur seuil nsl et lorsque la valeur n2 est inférieure ou égale à la valeur seuil ns2.

L'échantillon peut être de diverses origines, par exemple d'origine alimentaire, environnementale, vétérinaire, clinique, pharmaceutique ou cosmétique.

Parmi les échantillons d'origine alimentaire, l'on peut citer, de façon non-exhaustive, un échantillon de produits lactés (yaourts, fromages, etc.), de viande, de poisson, d'oeuf, de fruit, de légume, d'eau, de boisson (lait, jus de fruits, soda, etc.). Bien évidemment, ces échantillons d'origine alimentaire peuvent aussi provenir de sauces ou de plats plus élaborés ou des matières premières non transformées ou partiellement transformées. Un échantillon d'origine alimentaire peut également être issu d'une alimentation destinée aux animaux, telle que des tourteaux, des farines animales. Tel qu'indiqué précédemment, l'échantillon peut être d'origine environnementale et peut consister, par exemple, en un prélèvement de surface, d'eau, d'air, etc.

L'échantillon peut également consister en un échantillon d'origine clinique, pouvant correspondre à des prélèvements de fluide biologique (urine, sang total, ou dérivés tel que sérum, salive, pus, liquide céphalo-rachidien, etc.), de selles (par exemple diarrhées cholériques), de prélèvements de nez, de gorge, de peau, de plaies, d'organes, de tissus ou de cellules isolées. Cette liste n'est évidemment pas exhaustive. De manière préférée l'échantillon est d'origine pharmaceutique, et correspond par exemple à des préparations pharmaceutiques ou à des préparations vaccinales.

De manière générale, le terme « échantillon » se réfère à une partie ou à une quantité, plus particulièrement une petite partie ou une petite quantité, prélevée à partir d'une ou plusieurs entités aux fins d'analyse. Cet échantillon peut éventuellement avoir subi un traitement préalable, impliquant par exemple des étapes de mélange, de dilution ou encore de broyage, en particulier si l'entité de départ est à l'état solide.

L'échantillon prélevé est, en général, susceptible de - ou suspecté de - contenir au moins un micro-organisme cible et principalement une bactérie.

Le terme « micro-organisme » a la même signification que celle généralement admise en microbiologie et comprend notamment les bactéries gram positif ou gram négatif, les levures, les moisissures et plus généralement, les organismes unicellulaires, invisibles à l'œil nu, qui peuvent être manipulés et multipliés en laboratoire.

Avantageusement, l'échantillon est mis en contact avec au moins un milieu de culture permettant la croissance des micro-organismes et, en particulier du ou des micro-organismes cibles. Par « milieu de culture », l'on entend un milieu comprenant tous les éléments nécessaires à la survie et/ou à la croissance des micro-organismes et, en particulier des micro-organismes recherchés (par exemple de l'eau tamponnée peptonée). Le milieu de culture peut contenir d'éventuels additifs, par exemple : des peptones, un ou plusieurs facteurs de croissance, des hydrates de carbone, un ou plusieurs agents sélectifs, des tampons, une ou plusieurs vitamines, etc.

Au sein de la présente invention, le terme « faisceau » utilisé notamment dans les expressions « faisceau lumineux d'excitation » ou « faisceau lumineux de réaction » désigne un ou plusieurs faisceaux comprenant un ou plusieurs rayons lumineux.

### Brève description des dessins

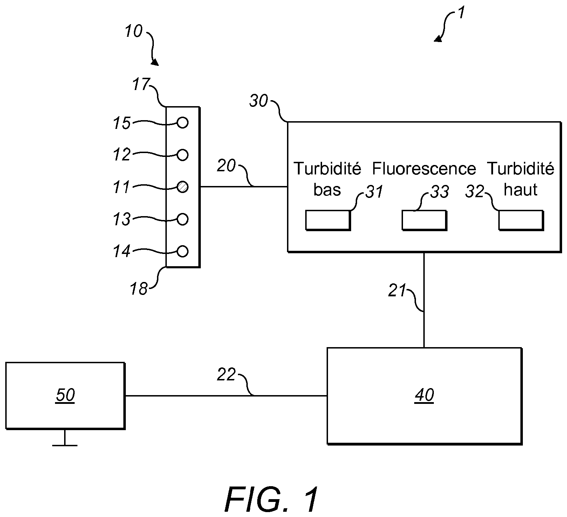

L'invention apparaîtra plus clairement à la lecture de la description qui suit, faite en référence aux figures correspondantes, et représentant, à titre d'exemple non limitatif, un procédé et un système selon la présente invention. Plus précisément : la figure 1 montre, de façon schématique, un système de détection comprenant un dispositif de détection selon un mode de réalisation de l'invention ;
la figure 2 représente un exemple non conforme à l'invention, fixé sur la paroi externe d'un contenant d'un premier diamètre, positionné de façon essentiellement verticale, selon un premier mode de réalisation de l'invention ;
la figure 3 montre le dispositif de détection, selon la figure 2, fixé sur un contenant d'un deuxième diamètre ;
la figure 4 représente le dispositif de détection, selon les figures 2 et 3, utilisé avec le contenant d'un premier diamètre positionné de façon essentiellement horizontale ;
la figure 5 montre le dispositif de détection selon un deuxième exemple non conforme à l'invention ;
la figure 6 représente le dispositif de détection, selon la figure 5, utilisé avec le contenant d'un premier diamètre positionné de façon essentiellement horizontale ;
la figure 7 montre le système de détection selon l'invention ;
la figure 8 représente des résultats obtenus en utilisant un système de détection selon un mode de réalisation de l'invention, lesdits résultats comprenant un premier signal de réaction concernant un premier paramètre d'analyse associé à la disparition de la fluorescence au sein du contenant et un deuxième signal de réaction concernant un deuxième paramètre d'analyse associé à la présence de turbidité au sein du contenant ;

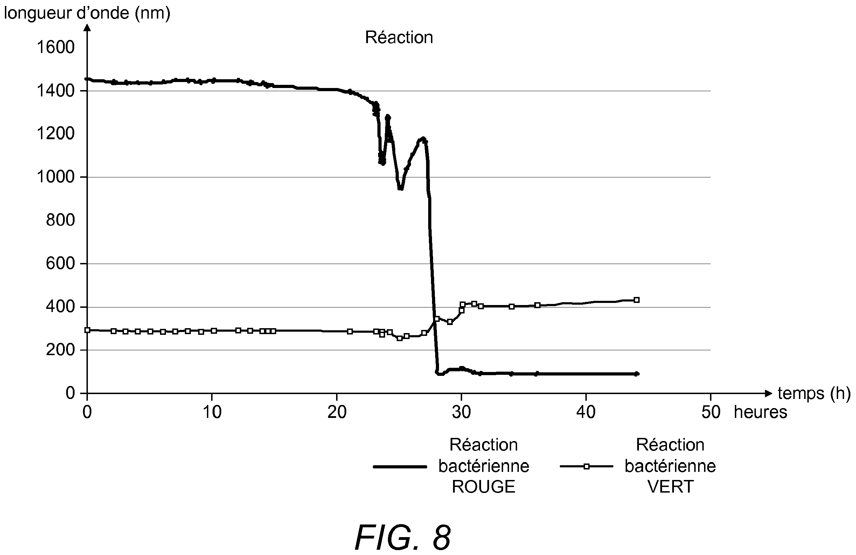

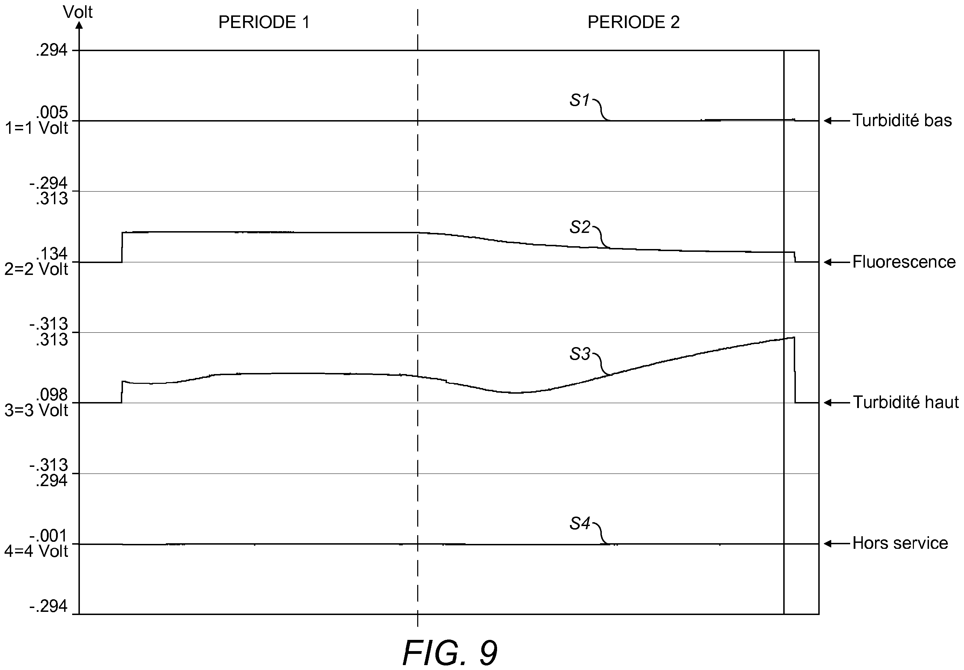

la figure 9 représente des résultats obtenus en utilisant un système de détection selon une alternative au mode de réalisation de l'invention de la figure 8, lesdits résultats comprenant notamment un premier signal de réaction concernant un premier paramètre d'analyse associé à la présence de turbidité au sein du contenant en position basse du contenant, un deuxième signal de réaction concernant la disparition de la fluorescence au sein du contenant et un troisième signal de réaction concernant le premier paramètre d'analyse associé à la présence de turbidité au sein du contenant en position haute du contenant.

### Description détaillée de l'invention

La figure 1 montre, de façon schématique, les différents éléments d'un premier exemple d'un système de détection 1. Le système de détection 1 comprend un dispositif de détection 10, adapté pour être fixé sur la paroi externe d'un contenant, dont les fonctionnalités sont décrites en détail ci-dessous.

Au sein du système de détection 1, le contenant contient notamment un milieu de culture et un échantillon à observer. De manière alternative, l'échantillon peut être mis en contact avant d'être introduit dans le contenant.

Le contenant est adapté pour permettre l'application du procédé de détection selon l'invention et notamment l'observation, à l'intérieur du contenant, d'une éventuelle croissance microbienne. Ainsi, le contenant selon l'invention est au moins partiellement translucide et comprend donc une paroi avec au moins une zone translucide. Le caractère translucide de la paroi du contenant est nécessaire pour laisser passer un faisceau lumineux d'excitation, de l'extérieur du contenant vers l'intérieur du contenant. Comme décrit ci-dessous, le faisceau lumineux de réaction, généré à l'intérieur du contenant en réponse au faisceau lumineux d'excitation, doit être détecté afin d'être capturé et mesuré par un capteur, tel qu'un photodétecteur, de type photodiode, à l'extérieur dudit contenant. Le caractère translucide de la paroi du contenant utilisé dans le procédé de détection, et au sein du système de détection 1, peut résulter de l'utilisation de matières telle que du verre ou du plastique dans la fabrication dudit contenant. De manière avantageuse, le contenant selon l'invention comprend une paroi avec au moins une zone transparente.

Comme montré sur la figure 1, au sein du système de détection 1, le dispositif de détection 10 est connecté, à l'aide d'une première connexion filaire 20, à un convertisseur de signal 30 afin de permettre la conversion d'un signal analogique en un signal numérique. Ainsi, le convertisseur de signal 30 permet de convertir tout signal analogique détecté par le dispositif de détection 10 en un signal numérique aux fins d'analyse. Le convertisseur de signal 30 est commercialisé, par exemple, sous la forme d'un coffret de lecture par DATAQ^{™} Instruments, Inc. sous la référence DI-7188. Le convertisseur de signal 30 comprend, par exemple, trois unités de conversion 31, 32 et 33. Chaque unité de conversion 31, 32 et 33 permet la conversion d'un signal analogique spécifique détecté au moyen du dispositif de détection 10. Ainsi, la première unité de conversion 31 permet, par exemple, de convertir le signal analogique associé à la présence ou à l'absence de turbidité en un premier endroit précis à l'intérieur du contenant en observation. Une deuxième unité de conversion 32 permet, par exemple, de convertir le signal analogique associé à la présence ou à l'absence de turbidité en un deuxième endroit précis à l'intérieur du contenant en observation. Une troisième unité de conversion 33 permet, par exemple, de convertir le signal analogique associé à la présence ou à l'absence de fluorescence du fluide observé. Le convertisseur de signal 30 est connecté, à l'aide d'une connexion filaire 21, à une source d'alimentation 40. La source d'alimentation 40 comprend par exemple des piles telles que deux piles Li, format AA (2600mAh). L'ensemble composé du dispositif de détection 10, du convertisseur de signal 30 et de la source d'alimentation 40, est lui-même connecté à un dispositif de contrôle 50, tel qu'un ordinateur, au moyen d'une connexion filaire 22.

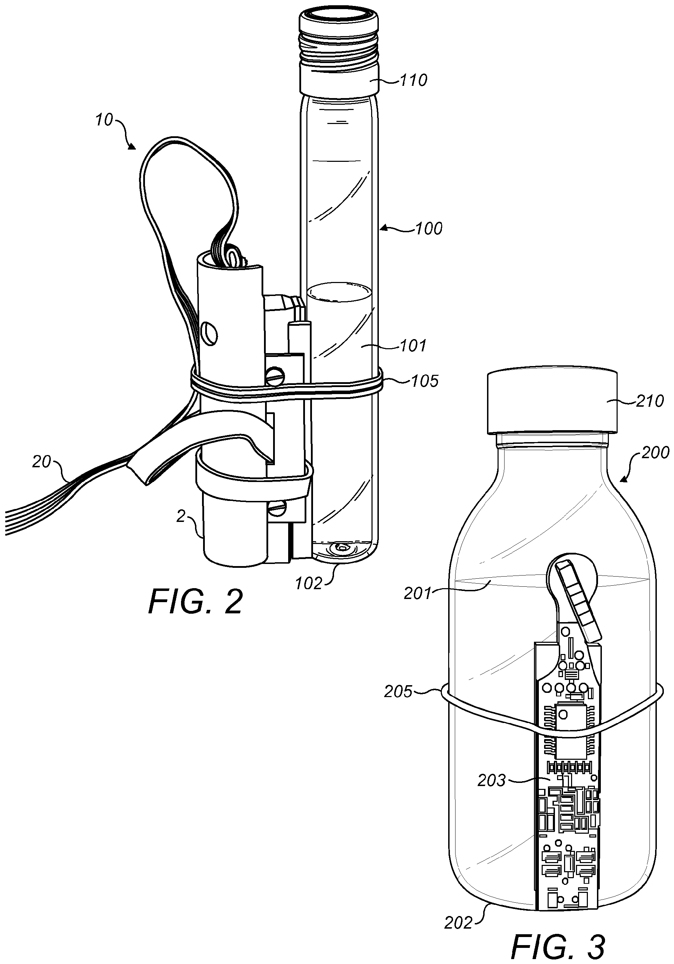

Le dispositif de contrôle 50 permet de réaliser différentes fonctions. Les fonctions consistent notamment à traiter, analyser et comparer les différents signaux numériques transmis par le convertisseur de signal 30 et issus de la détection de faisceaux lumineux de réaction, comme décrit ci-après, au moyen du dispositif de détection 10. Le traitement des signaux numériques comprend notamment la réception et la mise en mémoire des signaux numériques associés à la détection des faisceaux lumineux de réaction. Une autre fonction du dispositif de contrôle 50 consiste à régler les paramètres techniques des différents éléments présents dans le dispositif de détection 10 lors de l'utilisation du système 1 selon la figure 1. Ces paramètres techniques comprennent, par exemple, des valeurs seuil déterminées concernant la présence ou l'absence de turbidité et la présence ou l'absence de fluorescence. Ainsi, selon la valeur du signal détecté lors du procédé d'analyse de l'échantillon, un signal d'alerte peut être émis, de manière passive pour un utilisateur, afin d'indiquer à l'utilisateur la présence d'une contamination microbiologique selon que la valeur correspondant au signal détecté est inférieure ou supérieure à la valeur seuil concernée. Aucune surveillance active des signaux, par un utilisateur, n'est donc requise. Le dispositif de détection 10 est adapté, comme montré sur les figures 2, 3, 4, 5 et 7, pour être fixé sur la paroi externe d'un contenant 100, 200 et pour permettre d'observer des modifications relatives au contenu à l'intérieur dudit contenant 100, 200. La nature et l'emplacement des différents éléments du dispositif de détection 10 selon l'invention permettent d'optimiser le suivi de la transformation du contenu liée à une éventuelle croissance microbienne se développant dans le contenu du contenant 100, 200.

Le dispositif de détection 10 est pourvu d'une source lumineuse 11 produisant un faisceau lumineux d'excitation. La source lumineuse 11 comprend, par exemple, une diode électroluminescente (LED) d'un diamètre de 3mm. Une LED de ce type peut représenter un spectre centré autour de 557nm, avec une largeur à mi-hauteur de 22nm. Une telle LED est commercialisée, par exemple, par SIEMENS^{™} sous la référence LP3440. Un module électronique (non montré), tel qu'un circuit électronique d'excitation à courant constant, peut être accolé au dispositif de détection 10 pour assurer la fonction de pilotage de la source lumineuse 11 et notamment le paramétrage des paramètres relatifs à l'émission du faisceau lumineux d'excitation, tels que l'intensité ou la durée d'émission de la source lumineuse. Le courant constant appliqué est, par exemple, de 15,6mA et l'amplification des photo-courant est telle que U=109 I où I représente l'intensité. Ce module électronique peut également être intégré au dispositif de contrôle 50.

Le dispositif de détection 10 est également pourvu d'au moins un moyen de détection tel qu'un capteur de type photodétecteur. Le moyen de détection comprend un premier photodétecteur 12, telle qu'une photodiode 12, qui est spécifiquement utilisé pour l'observation d'un premier paramètre d'analyse tel que la fluorescence d'un fluide à l'intérieur du contenant 100, 200. La photodiode 12 est équipée d'un filtre « rouge » afin de permettre l'observation de la fluorescence d'un fluide à l'intérieur du contenant 100, 200. Par exemple, la photodiode 12 est une photodiode à large spectre filtrée dans le rouge au moyen d'un filtre dit « filtre dichroïque passe bande » présentant un spectre supérieur à 610nm. La photodiode 12 est caractérisée par un angle de vision relativement fermé, à +/- 15°, et un faible courant d'obscurité à 20pA. Ce type de photodiode est commercialisé, par exemple, par PERKIN ELMER sous la référence VTB 1113. La source lumineuse 11 permet la transmission d'une certaine quantité de lumière vers l'intérieur du contenant 100, 200. En présence d'une croissance microbienne, un faisceau lumineux de réaction généré à l'intérieur du contenu du contenant 100, 200 peut être capturé à l'aide de la première photodiode 12. Comme indiqué en détail ci-dessous, la détection de la fluorescence émise par un fluide représente une première possibilité d'observer une éventuelle croissance microbienne au sein du fluide localisé à l'intérieur du contenant 100, 200.

Le moyen de détection comprend un deuxième photodétecteur 13, ou photodiode 13, utilisé pour observer la turbidité du contenu du contenant. La photodiode 13 est équipée d'un filtre « vert » afin de permettre l'observation d'un deuxième paramètre d'analyse tel que la turbidité du fluide. Par exemple, la photodiode 13 est une photodiode à large spectre filtrée dans le vert au moyen d'un filtre dit « filtre dichroïque passe-bande », présentant un spectre de 500 à 570nm. La photodiode 13 est caractérisée par un angle de vision relativement fermé, à +/- 15°, et un faible courant d'obscurité à 20pA. Ce type de photodiode est commercialisé, par exemple, par PERKIN ELMER sous la référence VTB 1113. La quantité de lumière issue du faisceau lumineux de réaction et emmagasinée par la photodiode 13 équipée du filtre « vert » indique l'éventuelle apparition d'un trouble dans le contenu du contenant 100, 200. Le trouble est associé, par exemple, à la présence de particules solides au sein du fluide. La détection de la turbidité représente une deuxième possibilité pour constater une éventuelle croissance microbienne au sein du fluide localisé à l'intérieur du contenant 100, 200.

La mesure de la turbidité à l'aide d'une source lumineuse et d'au moins un moyen de détection tels que des photodétecteurs est liée au fait que lorsque de la matière est exposée à des rayons électromagnétiques, ces rayons électromagnétiques interagissent avec les charges électroniques des atomes. Une partie des rayons traverse la matière sans que la direction des rayons ne soit modifiée. Une autre partie des rayons est diffusée dans toutes les directions. Cela signifie que chaque particule illuminée se comporte comme une source lumineuse ponctuelle. Pour cette raison, la quantité de lumière diffusée par les particules présentes à l'intérieur d'un fluide augmente avec la quantité et la dimension desdites particules. De plus, lorsque ces particules sont des micro-organismes, l'on constate que la diffusion de lumière n'apparaît pas de façon homogène dans toutes les directions, mais de façon prédominante dans une direction proche des rayons lumineux émis par une source lumineuse. Ainsi, la disposition de la source lumineuse et d'au moins un moyen de détection du même côté à l'extérieur du contenant 100, 200 et du même côté de la paroi du contenant, avec une distance déterminée entre la source lumineuse et le moyen de détection, permet d'optimiser la détection de la turbidité au sein du contenu du contenant 100, 200 comme détaillé ci-après. Comme indiqué sur la figure 1, le dispositif de détection 10 est pourvu d'une extrémité supérieure 17 et d'une extrémité inférieure 18. Le moyen de détection est pourvu d'un troisième photodétecteur 14, ou troisième photodiode 14, positionné relativement près de l'extrémité inférieure 18 du dispositif de détection 10. La photodiode 14 est, par exemple, filtrée dans le vert. La photodiode 14 peut être adaptée pour observer des événements à un endroit spécifique du contenant 100, 200, telle qu'une croissance microbienne localisée à proximité du fond du contenant 100, 200 lorsque le dispositif de détection 10 est fixé sur ledit contenant 100, 200. Le moyen de détection peut être pourvu d'un quatrième photodétecteur 15, ou quatrième photodiode 15, adapté pour une détection spécifique en fonction du fluide observé au moyen du dispositif de détection 10. Comme indiqué précédemment, la source lumineuse 11 est utilisée pour diriger, à l'intérieur du contenant 100, 200, une certaine quantité de lumière ou faisceau lumineux d'excitation dans le but d'entraîner la génération d'un faisceau lumineux de réaction à l'intérieur du contenu présent dans le contenant 100, 200. Le moyen de détection 12, 13, 14, 15 permet donc de détecter au moins un signal lumineux de réaction émis par le contenu du contenant 100, 200, afin de suivre une éventuelle croissance microbienne à l'intérieur du contenant 100, 200 pour déterminer la contamination microbiologique de l'échantillon liée à la présence d'au moins un micro-organisme. De manière générale, les différents photodétecteurs 12 à 15 du moyen de détection disposent notamment d'une fonction permettant d'ajouter des filtres afin de pouvoir recevoir des faisceaux lumineux présentant une longueur d'onde spécifique.

Le fonctionnement du système de détection 1 selon la figure 1 est décrit ci-dessous en faisant référence aux figures 2, 3 et 4.

La figure 2, 3 et 4 montrent un contenant 100, 200, muni d'un moyen d'obturation 110, 210 tel qu'un bouchon, ledit contenant 100, 200 contenant un contenu 101, 201 tel qu'un fluide. Le contenu 101, 201 comprend un milieu de culture adapté pour optimiser la croissance microbienne. Sur la figure 3, le contenant 200 présente un diamètre supérieur au diamètre du contenant 100 selon la figure 2. Comme montrés sur les figures 2, 3 et 4, le dispositif de détection 10 est positionné et connecté à l'extérieur du contenant 100, 200. Cela signifie que l'on peut observer la croissance microbienne à l'intérieur du contenant 100, 200 et ce, sans être contraint d'introduire un moyen d'observation à l'intérieur dudit contenant 100, 200.

Comme montré sur les figures 2, 3 et 4, le dispositif de détection 10 est fixé sur l'extérieur du contenant 100, 200, de sorte que ledit dispositif de détection 10 détecte une éventuelle croissance microbienne au sein du contenu 101, 201 du contenant 100, 200. Le dispositif de détection 10 est fixé sur la paroi externe du contenant 100, 200 à l'aide de tout moyen de connexion 105, 205 adapté, par exemple à l'aide d'une bande élastique. Le moyen de connexion 105, 205 permet de connecter le dispositif de détection 10 directement sur la paroi du contenant 100, 200 afin que la distance entre le dispositif de détection 10 et la paroi du contenant 100, 200 soit la plus faible possible. Le moyen de connexion 105, 205 peut également permettre de connecter le moyen de détection 10 en conservant une distance déterminée avec la paroi du contenant 100, 200. La distance déterminée correspond à un espace libre, de l'ordre de quelques centimètres, afin que le dispositif de détection 10 et la paroi du contenant 100, 200 ne soient pas en contact. Le dispositif de détection 10 selon l'invention présente donc l'avantage d'être connecté à l'extérieur du contenant 100, 200 sur la paroi du contenant 100, 200 ou à proximité de la paroi du contenant 100, 200, quelle que soit la forme dudit contenant 100, 200 et ce à l'aide du moyen de connexion 105, 205. Cet avantage permet d'utiliser le dispositif de détection 10 lors de l'observation d'une croissance microbienne à l'intérieur de n'importe quel type de contenant, c'est-à-dire quelle que soit la forme et la taille du contenant.

Ainsi, contrairement aux procédés divulgués dans l'art antérieur, lorsque le MFT est finalisé, il n'est pas nécessaire de transférer le contenu 101, 201 du contenant 100, 200 comprenant l'échantillon biologique, dans un autre contenant spécifiquement adapté pour être utilisé dans un dispositif d'analyse particulier. Dans la suite de la description des figures 2, 3 et 4, l'on fait référence à l'utilisation du système de détection 1 selon l'invention, en particulier pour analyser le contenu d'un contenant 100, 200 obtenu à la fin d'un test de type « Media-Fill-Test » (MFT). Au cours de l'utilisation d'un dispositif de détection 10, tel que montré sur la figure 2, dès l'introduction dudit échantillon au sein du contenu 101, la croissance microbienne peut être observée à l'aide du système de détection 1 selon l'invention.

De manière générale, en référence aux figures 2, 3, 4 et 6, la source lumineuse 11 et le moyen de détection 12, 13, 14, 15 sont disposés à l'extérieur du contenant 100, 200. Afin d'optimiser la détection du faisceaux lumineux de réaction, en provenance du contenu 101 du contenant 100, 200, par le moyen de détection 12, 13, 14, 15, la source lumineuse 11 et le moyen de détection 12, 13, 14, 15 doivent être disposés au niveau de la zone translucide du contenant 100, 200, c'est-à-dire à une distance suffisamment proche de la zone translucide pour permettre au faisceau lumineux d'excitation émis par la source lumineuse 11 d'être émis au travers de la zone translucide et au faisceau lumineux de réaction en provenance du contenu du contenant 100, 200 d'être détecté par le moyen de détection 12, 13, 14, 15. Ainsi, la source lumineuse 11 et le moyen de détection 12, 13, 14, 15 sont localisés au niveau de la zone translucide de la paroi du contenant 100, 200. Le moyen de détection 12, 13, 14, 15 est positionné selon un angle de valeur déterminée par rapport à la direction du faisceau lumineux d'excitation. Ainsi, le moyen de détection 12, 13, 14, 15 peut détecter de manière optimale le faisceau lumineux de réaction généré par le contenu 101 du contenant 100, 200. Les valeurs déterminées de l'angle sont comprises entre 0° et 180°. De manière avantageuse, les valeurs déterminées de l'angle sont comprises entre 0° et 90°. De manière particulièrement avantageuse, la valeur déterminée de l'angle est comprise entre 0° et 30°. La valeur de l'angle peut également être sensiblement égale à 0°. De plus, la distance entre la source lumineuse 11 et le moyen de détection 12, 13, 14, 15 est généralement de l'ordre de quelques centimètres afin de garantir une relative proximité entre la source lumineuse 11 et le moyen de détection 12, 13, 14, 15 lors du fonctionnement du dispositif de détection 10. Au sein du dispositif de détection 10 tel que montré sur la figure 2, la source lumineuse 11 et le moyen de détection 12, 13, 14 et 15, montrés en figure 1, sont disposés, de manière avantageuse, afin d'être alignés à l'extérieur du contenant 100 dès lors que le dispositif de détection 10 est fixé sur la paroi du contenant 100.

Lors de l'utilisation de l'exemple non conforme à l'invention, tel que montré dans la figure 2, le contenant 100 est utilisé, dans une position verticale, le fond 102 dudit contenant 100 étant lui- même posé sur un support. Le dispositif de détection 10 selon l'invention est fixé sur l'extérieur du contenant 100 afin d'être positionné le plus près possible du fond 102 du contenant 100. Dans cette position, la photodiode 14, positionnée le plus bas possible, comme montré sur la figure 1, est spécifiquement adaptée pour permettre le suivi d'une éventuelle croissance microbienne à l'intérieur du contenant 100, près du fond 102. Un cache de protection 2 peut être ajouté afin de protéger les différents éléments du dispositif de détection 10 de tout éventuel endommagement.

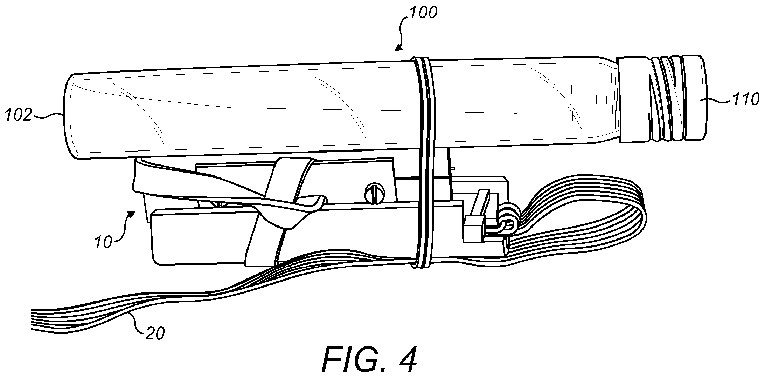

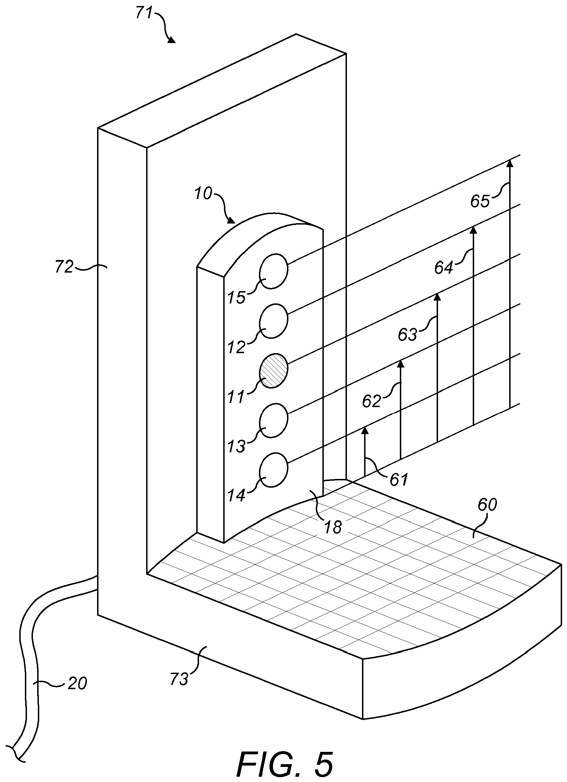

Le dispositif de détection 10 selon l'exemple non conforme à l'invention est représenté sur la figure 3, dépourvu du cache de protection 2 montré sur la figure 2 afin de montrer la partie intérieure du dispositif de détection 10 qui comprend, notamment, une carte électronique 203 pourvue de différents composants électroniques. La carte électronique 203 est adaptée pour recevoir des instructions transmises depuis le dispositif de contrôle 50, montré sur la figure 1, et transmettre ces instructions vers différents éléments, notamment vers la source lumineuse 11 et le moyen de détection 12, 13, 14 et 15, grâce aux connexions filaires 20, 21 et 22. La figure 4 décrit un l'exemple non conforme à l'invention dans lequel le contenant 100, selon la figure 2, est utilisé dans une position essentiellement horizontale, le moyen d'obturation 110 et le fond 102 étant positionnés essentiellement sur un même plan horizontal. La position particulière du contenant 100 permet, par exemple, de détecter la présence de germes, en observant la présence d'une couche de sédimentation de ces germes. Selon un troisième l'exemple non conforme à l'invention, la figure 5 montre un dispositif de support 70 comprenant un socle 71 en forme de « L », présentant une première partie 72 et une deuxième partie 73 comprenant une surface 60. Comme représenté sur la figure 5, le dispositif de détection 10 est intégré à la première partie 72.

Un contenant de n'importe quelle taille ou dimension peut être positionné sur la surface 60 de la deuxième partie 73 du socle 71 afin que la surface du fond du contenant se superpose à la surface 60. Ainsi, en utilisant le dispositif de détection 10 selon la figure 5, la distance entre le fond du contenant et les éléments 11, 12, 13, 14 et 15 représente une distance fixe. Sur la figure 5, la distance fixe est indiquée à l'aide des flèches 61 à 65. La distance fixe permet de repérer avec précision l'emplacement d'un contenant afin de placer un autre contenant dans les mêmes conditions de test lors de l'analyse du contenu de cet autre contenant. Le socle 71 permet donc de reproduire avec précision des conditions particulières relatives au test et ce, quel que soit le type de contenant.

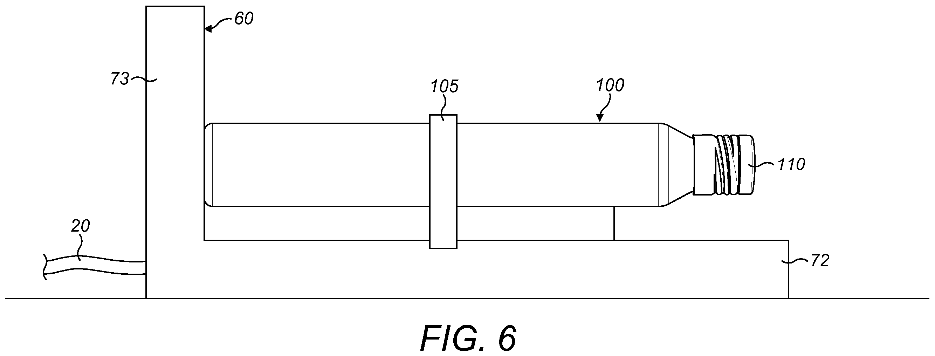

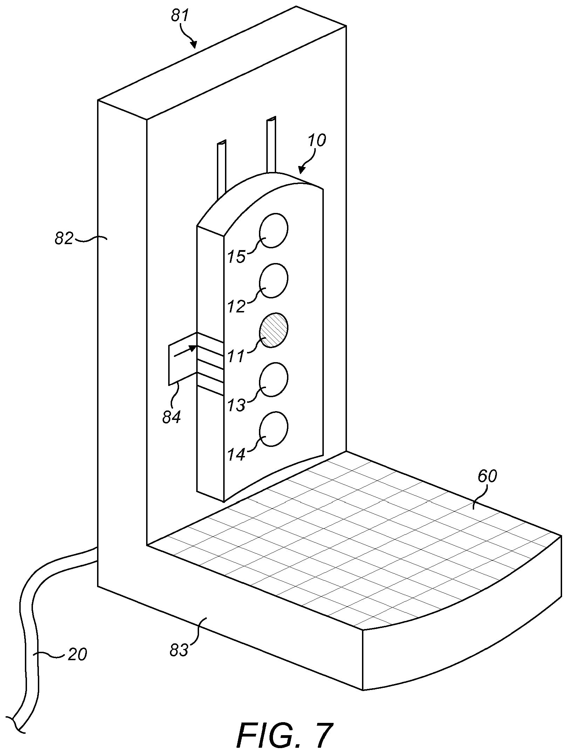

La figure 6 représente le dispositif de support selon la figure 5 utilisé en combinaison avec un contenant 100, ledit contenant 100 étant disposé dans une position essentiellement horizontale. Le fond du contenant 100 est en contact avec la surface 60 de la deuxième partie 73 du socle 71. La position fixe du contenant 100 sur le dispositif est assurée grâce au moyen de connexion 105. Dans cette position,

l'utilisateur peut aisément détecter le dépôt éventuel de particules solides associées à l'existence d'une turbidité du fluide. Selon le mode de réalisation de l'invention, la figure 7 montre un dispositif de support comprenant un socle 81, comportant une première partie 82 et une deuxième partie 83. Le dispositif de détection 10 est fixé sur la première partie 82 du socle 81. Selon la figure 7, la distance entre le dispositif de détection 10 et la surface 60 est réglable. Cela signifie que le dispositif de détection 10 peut être disposé dans une position adaptée référencée à l'aide d'un indicateur 84 avant de positionner un contenant sur la surface 60. Le dispositif de support selon la figure 7 étant réglable, l'utilisateur peut aisément adapter les contraintes liées à la forme du contenant pour optimiser le déroulement du test.

Le système de détection 1 fonctionne selon un procédé de détection comprenant les étapes suivantes. Ainsi, une première étape comprend l'introduction d'un échantillon dans un contenant 100, 200 comprenant une paroi avec au moins une zone translucide. L'échantillon est susceptible de contenir au moins un micro-organisme. L'échantillon est donc mis en contact avec un milieu de culture adapté pour permettre la croissance du micro-organisme. Le mélange de l'échantillon et dudit milieu de culture forme tout ou partie du contenu 101, 201 du contenant 100, 200. La mise en contact de l'échantillon avec le milieu de culture peut avoir lieu soit préalablement à l'introduction de l'échantillon au sein du contenant 100, 200, soit après l'introduction de l'échantillon au sein du contenant 100, 200.

Le procédé de détection comprend une deuxième étape optionnelle comprenant, l'incubation du contenant 100, 200 à une température et durant un laps de temps suffisants pour permettre la croissance dudit au moins un micro-organisme. De manière avantageuse, le procédé de détection comprend cette étape d'incubation.

Une troisième étape comprend l'illumination du contenu du contenant 100, 200 au travers de la zone translucide avec la source lumineuse 11 qui émet un faisceau lumineux d'excitation.

Une quatrième étape comprend la détection à l'aide du moyen de détection 12, 13, 14, 15 d'au moins un faisceau lumineux de réaction émis en réponse à l'illumination du contenu, c'est-à-dire à l'émission du faisceau lumineux d'excitation par la source lumineuse 11. Le faisceau lumineux de réaction est associé à une détection par mesure d'un paramètre d'analyse représentatif d'une éventuelle présence microbienne à l'intérieur du contenant 100, 200. Le choix du paramètre d'analyse dépend du type de détection souhaité. Ainsi, un premier paramètre d'analyse peut concerner la turbidité et un deuxième paramètre d'analyse peut concerner la fluorescence.

Dans une cinquième étape, le dispositif de détection 10 reçoit et transmet au convertisseur de signal 30 le faisceau lumineux de réaction pour une conversion en un signal numérique de réaction. Ainsi, le convertisseur de signal 30 permet d'obtenir une valeur n associée au paramètre d'analyse considéré. Chaque valeur n est ainsi mesurée périodiquement au cours d'une durée spécifique et est associée à un faisceau lumineux de réaction détecté par le moyen de détection 12, 13, 14, 15.

Lorsque le paramètre d'analyse considéré est la turbidité, les différentes valeurs n mesurées croissent lorsque la quantité du micro-organisme au sein du contenu du contenant 100, 200 croît. Lorsque le paramètre d'analyse considéré est la fluorescence, les différentes valeurs n mesurées peuvent croître ou décroître lorsque la quantité du micro-organisme au sein du contenu du contenant 100, 200 croît, selon la nature du micro-organisme.

Une sixième étape comprend la comparaison de la valeur n mesurée avec une valeur seuil ns du même paramètre d'analyse. La valeur seuil ns est indicative de la présence d'au moins un micro-organisme dans l'échantillon et est associée au paramètre d'analyse considéré.

Dans une septième étape, selon le résultat de la comparaison entre les valeurs n et ns, l'on peut déduire la présence ou l'absence d'au moins un micro-organisme, au sein de l'échantillon.

Ainsi, lorsque le paramètre d'analyse concerne la turbidité, la présence du microorganisme au sein du contenu du contenant 100, 200 est avérée lorsque la valeur n mesurée est supérieure ou égale à la valeur seuil ns pour ce même paramètre d'analyse relatif à la turbidité. Lorsque le paramètre d'analyse concerne la fluorescence, la présence du microorganisme au sein du contenu du contenant 100, 200 est avérée lorsque la valeur n mesurée est supérieure ou égale à la valeur seuil ns pour le même paramètre d'analyse ou lorsque la valeur n mesurée est inférieure ou égale à la valeur seuil ns pour ce même paramètre d'analyse relatif à la fluorescence. La condition de croissance ou de décroissance de la valeur n mesurée de la fluorescence dépend de la nature du micro-organisme recherché.

Le procédé de détection peut comprendre une combinaison d'étapes de détection relatives à plusieurs paramètres d'analyse, de manière simultanée.

Ainsi, le procédé de détection permet, selon un mode de réalisation alternatif, de mesurer des valeurs n associées à un premier et à un deuxième paramètre d'analyse. Ainsi, les valeurs n mesurées sont comparées avec les valeurs seuil respectives nsl et ns2 de chaque paramètre d'analyse. Selon le résultat de l'étape de comparaison précédemment décrite, l'on peut déduire la présence ou l'absence d'un micro-organisme au sein du contenu 101, 201 du contenant 100, 200.

Cette combinaison des paramètres d'analyse pour détecter la présence d'un micro- organisme au sein du contenu du contenant 100, 200 permet de valider de manière fiable la présence ou l'absence d'un micro-organisme.

La figure 8 représente les résultats de l'observation d'un récipient 100 contenant un fluide 101 comprenant un échantillon biologique.

Le dispositif de détection 10 selon l'invention est utilisé pour mettre en évidence une éventuelle croissance microbienne à l'intérieur d'un contenant 100 selon un premier paramètre d'analyse associé à la disparition de la fluorescence au sein du contenant 100.

Comme représenté sur la figure 8, après une première période d'incubation de l'ordre de 24 heures, l'on peut constater, concernant le photodétecteur 12 associé à la réaction bactérienne rouge, le début de l'évolution d'une croissance microbienne laissant apparaître une oscillation de période de 1 à 2mn et d'amplitude de 2 à 3 % du niveau avec une valeur moyenne en décroissance de l'ordre de 10 % par heure. On peut constater une chute brutale de la réponse, c'est-à-dire de la fluorescence à l'intérieur du contenant, après une période d'observation d'environ 27 à 28 heures.

Sur la figure 8, le signal obtenu grâce à la photodiode 12 montre une chute d'environ 1300 à 90nm. La photodiode 12 permet donc d'observer une disparition quasi complète de la fluorescence présente à l'intérieur du contenu 101.

Au cours de la même observation, le dispositif de détection est également utilisé pour mettre en évidence une éventuelle croissance microbienne à l'intérieur d'un contenant 100 selon un deuxième paramètre d'analyse associé à la présence de turbidité au sein du contenant. En effet, le photodétecteur 13, équipé d'un filtre vert, est utilisé pour suivre la réaction bactérienne « verte », ce qui signifie que l'on peut observer la turbidité présente à l'intérieur du contenant 100, à l'aide dudit photodétecteur 13. Après une période d'incubation de l'ordre de 24 heures, une augmentation d'intensité du signal est repérable sur la figure 8. Cette augmentation de l'intensité du signal, observée grâce au photodétecteur 13, indique la présence de particules au sein du fluide 101.

Comme décrit ci-dessus et comme représenté sur la figure 8, le système 1 selon l'invention permet de connecter un dispositif de détection 10 à l'extérieur d'un contenant 100, ledit dispositif de détection 10 permettant de suivre au moins un, voire de préférence, deux critères ou paramètres d'analyse. Comme montré sur la figure 8, tout événement survenant à l'intérieur du contenant 100 peut être suivi en continu. Les données obtenues lors de l'observation peuvent être stockées et comparées aux résultats d'autres tests du même type. Par ailleurs, les différents résultats peuvent faire l'objet d'analyses qui permettront de confirmer des conclusions préliminaires. Un exemple de test, effectué à l'aide du système 1 selon l'invention, est décrit en détail ci-dessous.

### Exemple

Les résultats obtenus grâce au test selon le présent exemple sont représentés sur la figure 9.

### Méthode

Milieu de culture « Media-Fill-Test » avec indicateur coloré : « BTS 3P irradié peptones Végétales avec Indicateur Coloré (MFTVCI-D) », référence 51104, bioMérieux,
Souche calibrée Bioball 550 CFU, Candida albicans ATCC 10231, référence 56013, bioMérieux,
Fluide de réhydratation pour Bioball, référence 56021, bioMérieux. Un tube en verre, rempli avec 10 ml de milieu de culture, est inoculé avec 55 CFU de Candida albicans (100 µḯ de suspension bactérienne Bioball). Le tube est fixé au dispositif de détection 10 à l'aide d'un élastique et mis dans une chambre noire en position horizontale, similaire aux positions montrées dans la figure 4, à température ambiante.

Comme indiqué sur la figure 9, quatre signaux SI, S2, S3 et S4 sont représentés au cours d'une période d'observation comprenant une période 1 et une période 2.

Le signal SI est associé à la détection d'une potentielle turbidité au sein du contenu du contenant en position basse du contenant. Comme indiqué sur la figure 9, le signal SI est constant pendant la durée totale d'observation. Ainsi, la représentation graphique du signal SI indique qu'aucune turbidité n'est présente au sein du contenu du contenant, en position basse dudit contenant au cours des périodes 1 et 2.

Le signal S2 est associé à la détection d'une potentielle fluorescence au sein du contenu du contenant. Comme indiqué sur la figure 9, le signal S2 est constant au cours de la période 1 et indique la présence d'une fluorescence au sein du contenant. Au cours de la période 2, le signal S2 décroît de manière régulière. Ainsi, la représentation graphique du signal S2 indique que le taux de fluorescence décroît au sein du contenant. Cette disparition de fluorescence permet d'indiquer que des micro-organismes sont présents au sein du contenant.

Le signal S3 est associé à la détection d'une potentielle turbidité au sein du contenu du contenant en position haute du contenant. Comme indiqué sur la figure 9, le signal S3 est quasiment constant pendant la période 1. Au cours de la période 2, le signal S3 décroît légèrement puis décroît plus fortement et enfin augmente de manière régulière. Les différents niveaux de décroissance sont associés à une mesure de la turbidité au sein du contenu du contenant à une période d'observation, la période 2, pendant laquelle la présence de micro-organismes est avérée. Cependant, au cours de cette période 2, les micro-organismes ne sont pas répartis, de manière homogène. Ainsi, le signal effectue des variations aléatoires avant de se stabiliser. Après une courte durée, l'augmentation du nombre de micro-organismes est telle que la répartition homogène des micro-organismes au sein du contenu du contenant permet d'obtenir un signal S3 qui croît régulièrement au fur et à mesure du temps.

Le signal S3 indique donc que la turbidité est présente au sein du contenant. La présence de la turbidité permet d'indiquer que des micro-organismes sont présents au sein du contenant. Le signal S4 est associé à un signal de référence correspondant à un état hors service du dispositif de détection 10. Ce signal S4 permet notamment de vérifier le bon fonctionnement des photodétecteurs par comparaison des valeurs numériques des signaux SI, S2 et S3 avec la valeur numérique de S4. Ainsi, en combinant l'observation des signaux S2 et S3, il apparaît que la présence de micro-organismes est confirmée à partir de la période 2 d'observation. Selon le paramétrage du dispositif de contrôle associé au dispositif de détection, une alarme peut être prévue pour avertir l'utilisateur de la présence d'une contamination microbiologique au sein du contenant.

## Revendications

1. Système de détection (1) comprenant un dispositif de détection (10) de la présence d'au moins un micro-organisme dans le contenu (101, 201) d'un contenant (100, 200) comprenant une paroi avec une zone translucide, ledit dispositif de détection (10) comprenant :
a) au moins une source lumineuse (11), telle qu'une diode électroluminescente (LED), susceptible d'illuminer le contenu (101, 201) du contenant (100, 200) en émettant un faisceau lumineux d'excitation au travers de la zone translucide du contenant (100, 200) ;
b) au moins un moyen de détection (12, 13, 14, 15), tel qu'une photodiode, pour détecter au moins un faisceau lumineux de réaction émis en réponse à l'illumination du contenu (101, 201) du contenant (100, 200) ; ledit au moins un moyen de détection (12, 13, 14, 15) étant positionné selon un angle de valeur déterminée par rapport à la direction du faisceau lumineux d'excitation pour détecter le faisceau lumineux de réaction; ladite au moins une source lumineuse (11) et ledit au moins un moyen de détection (12, 13, 14, 15) étant pourvus d'au moins un moyen de connexion (105, 205) entièrement localisé à l'extérieur du contenant (100, 200) pour connecter ladite au moins une source lumineuse (11) et ledit au moins un moyen de détection (12, 13, 14, 15) à la paroi du contenant (100, 200), au niveau de la zone translucide, ledit moyen de connexion (105, 205) permettant d'adapter la position du dispositif de détection (10) sur la paroi du contenant (100, 200),
c) un dispositif de contrôle (50) connecté à ladite au moins une source lumineuse (11) et audit au moins un moyen de détection (12, 13, 14, 15) pour contrôler le faisceau lumineux d'excitation émis par ladite au moins une source lumineuse (11) et/ou traiter et/ou analyser ledit au moins un faisceau lumineux de réaction émis en réponse à l'illumination du contenu du contenant (100, 200), et détecté par ledit au moins un moyen de détection (12, 13, 14, 15), **caractérisé en ce que** le système de détection comprend
d) un dispositif de support comprenant un socle (81) en forme de « L », comportant une première partie (82) et une deuxième partie (83) présentant une surface (60),
le dispositif de détection (10) étant fixé sur la première partie (82) du socle (81), la distance entre le dispositif de détection (10) et la surface (60) étant réglable.

2. Système de détection selon la revendication 1, dans lequel ledit au moins un moyen de détection (12, 13, 14, 15) comprend au moins un premier et un deuxième photodétecteur, respectivement positionnés à un premier et à un deuxième emplacement pour détecter, au niveau de la zone translucide du contenant (100, 200), un premier et un deuxième faisceau lumineux de réaction et ce, afin d'obtenir une première et une deuxième valeur d'un paramètre d'analyse représentatif d'une éventuelle présence microbienne dans le contenu (101, 201) du contenant (100, 200).

3. Système de détection selon la revendication 1, dans lequel ledit au moins un moyen de détection (12, 13, 14, 15) comprend un premier photodétecteur (12) adapté pour détecter un premier signal lumineux de réaction afin d'obtenir une valeur n d'un premier paramètre d'analyse représentatif d'une éventuelle présence microbienne dans le contenu du contenant (100, 200) et un deuxième photodétecteur (13) adapté pour détecter un deuxième faisceau lumineux de réaction, différent du premier faisceau lumineux de réaction, afin d'obtenir une valeur m d'un deuxième paramètre d'analyse représentatif d'une éventuelle présence microbienne dans le contenu (101, 201) du contenant (100, 200).

4. Système de détection selon la revendication 2 ou 3, dans lequel le premier photodétecteur (12) comprend une photodiode filtrée dans le rouge et le deuxième photodétecteur (13) comprend une photodiode filtrée dans le vert.

5. Système de détection (1) selon les revendications 1 à 4, dans lequel le dispositif de contrôle (50) comprenant :
a) un moyen de stockage pour stocker une première valeur du premier et du deuxième paramètre d'analyse obtenue avec ledit au moins un moyen de détection (12, 13, 14, 15) et une deuxième valeur du premier et du deuxième paramètre d'analyse obtenue après une durée de temps déterminée avec ledit au moins un moyen de détection (12, 13, 14, 15),
b) un moyen de comparaison pour comparer la première valeur et la deuxième valeur du premier et du deuxième paramètre d'analyse pour déterminer une éventuelle croissance microbienne dans le contenu du contenant (100, 200).

6. Système de détection (1) selon l'une des revendications 1 à 5, dans lequel le dispositif de contrôle (50) est adapté pour recevoir et stocker des valeurs obtenues avec ledit au moins un moyen de détection (12, 13, 14, 15), de manière continue.

7. Système de détection (1) selon l'une des revendications 1 à 6, dans lequel le dispositif de contrôle (50) comprend une alarme, pour indiquer une éventuelle présence microbienne dans le contenu (101, 201) du contenant (100, 200).

8. Procédé de détection de la présence d'au moins un micro-organisme dans un échantillon susceptible de contenir ledit au moins un micro-organisme, ledit procédé comprenant les étapes suivantes :
a) introduire l'échantillon dans un contenant (100, 200) comprenant une paroi avec au moins une zone translucide, ledit échantillon étant mis en contact avec un milieu de culture préalablement à l'introduction ou après l'introduction dudit échantillon au sein dudit contenant (100, 200), ledit au moins un milieu de culture étant adapté pour permettre la croissance dudit au moins un micro-organisme, le mélange de l'échantillon et dudit milieu de culture formant tout ou partie du contenu (101, 201) du contenant (100, 200),
b) éventuellement incuber le contenant (100, 200) à une température et durant un laps de temps suffisants pour permettre la croissance dudit au moins un micro-organisme,
c) mesurer, à l'aide d'un système de détection (1) selon l'une des revendications 1 à 7 au moins une valeur n d'au moins un paramètre d'analyse représentatif d'une éventuelle présence microbienne à l'intérieur du contenant (100, 200),
l'étape c) comprenant les sous-étapes consistant à :
i. illuminer le contenu (101, 201) du contenant (100, 200) au travers de la zone translucide avec la source lumineuse (11) et,
ii. détecter le faisceau lumineux de réaction émis en réponse à l'illumination du contenu (101, 201) avec ledit au moins un moyen de détection (12, 13, 14, 15) afin d'obtenir ladite valeur n du paramètre d'analyse,
d) comparer ladite valeur n avec une valeur seuil ns du même paramètre d'analyse, ladite valeur seuil ns étant indicative de la présence du au moins un micro-organisme dans l'échantillon, e) déduire la présence ou l'absence dudit au moins un micro-organisme, au sein de l'échantillon, à partir du résultat de la comparaison.

9. Procédé selon la revendication 8, dans lequel :
- l'étape c) comprend mesurer au moins un paramètre d'analyse dont la valeur n croît lorsque la quantité dudit micro-organisme croît, comme par exemple la turbidité,
- l'étape d) comprend comparer ladite valeur n avec une valeur seuil ns du même paramètre d'analyse, ladite valeur seuil ns étant indicative de la présence du au moins un micro-organisme dans l'échantillon,
- l'étape e) comprend déduire la contamination de l'échantillon par ledit au moins un micro-organisme lorsque la valeur n est supérieure ou égale à la valeur seuil ns.

10. Procédé selon la revendication 8, dans lequel :
- l'étape c) comprend mesurer au moins un paramètre d'analyse dont la valeur n décroît lorsque la quantité dudit micro-organisme décroît, comme par exemple la fluorescence,
- l'étape d) comprend comparer ladite valeur n avec une valeur seuil ns du même paramètre d'analyse,
- l'étape e) comprend déduire la contamination de l'échantillon par ledit au moins un micro-organisme lorsque la valeur n est inférieure ou égale à la valeur seuil ns.

11. Procédé selon la revendication 8, dans lequel :
- l'étape c) comprend mesurer au moins un premier paramètre d'analyse dont une valeur ni croît lorsque la quantité dudit micro-organisme croît, comme par exemple la turbidité et mesurer au moins un deuxième paramètre d'analyse dont une valeur n2 décroît lorsque la quantité dudit micro-organisme décroît, comme par exemple la fluorescence,
- l'étape d) comprend comparer ladite valeur ni avec une valeur seuil nsl associée au premier paramètre d'analyse et comparer ladite valeur n2 avec une valeur seuil ns2 associée au deuxième paramètre d'analyse,
- l'étape e) comprend la déduction de la contamination de l'échantillon par ledit au moins un micro-organisme lorsque la valeur ni est supérieure ou égale à la valeur seuil nsl et lorsque la valeur n2 est inférieure ou égale à la valeur seuil ns2.

## Patentansprüche

1. Nachweissystem (1), umfassend eine Vorrichtung zum Nachweis (10) der Anwesenheit mindestens eines Mikroorganismus im Inhalt (101, 201) eines Behälters (100, 200), der eine Wand mit einer lichtdurchlässigen Zone umfasst, wobei die Vorrichtung zum Nachweis (10) umfasst:
a) mindestens eine Lichtquelle (11) wie eine Leuchtdiode (LED), die geeignet ist, den Inhalt (101, 201) des Behälters (100, 200) zu beleuchten, indem sie einen Anregungslichtstrahl durch die lichtdurchlässige Zone des Behälters (100, 200) aussendet;
b) mindestens ein Nachweismittel (12, 13, 14, 15) wie eine Fotodiode zum Erfassen mindestens eines Reaktionslichtstrahls, der als Antwort auf die Beleuchtung des Inhalts (101, 201) des Behälters (100, 200) ausgesendet wird; wobei das mindestens eine Nachweismittel (12, 13, 14, 15) in einem Winkel mit einem Wert positioniert ist, der in Bezug auf die Richtung des Anregungslichtstrahls bestimmt wird, um den Reaktionslichtstrahl zu erfassen;
wobei die mindestens eine Lichtquelle (11) und das mindestens eine Nachweismittel (12, 13, 14, 15) mit mindestens einem Verbindungsmittel (105, 205) ausgestattet sind, das sich gänzlich außerhalb des Behälters (100, 200) befindet, um die mindestens eine Lichtquelle (11) und das mindestens eine Nachweismittel (12, 13, 14, 15) mit der Wand des Behälters (100, 200) an der lichtdurchlässigen Zone zu verbinden, wobei das Verbindungsmittel (105,205) es ermöglicht, die Position des Nachweismittels (10) an der Wand des Behälters (100, 200) anzupassen,
c) ein Steuermittel (50), das mit der mindestens einen Lichtquelle (11) und dem mindestens einen Nachweismittel (12, 13, 14, 15) verbunden ist, um den von der mindestens einen Lichtquelle (11) ausgesendeten Anregungslichtstrahl zu steuern und/oder den mindestens einen Reaktionslichtstrahl, der als Antwort auf die Beleuchtung des Inhalts des Behälters (100, 200) ausgesendet wird und von dem mindestens einen Nachweismittel (12, 13, 14, 15) erfasst wird, zu behandeln und/oder zu analysieren, **dadurch gekennzeichnet, dass** das Nachweissystem umfasst:
d) eine Trägervorrichtung, die einen L-förmigen Sockel (81) umfasst, der einen ersten Teil (82) und einen zweiten Teil (83), der eine Oberfläche (60) aufweist, beinhaltet,
wobei das Nachweismittel (10) am ersten Teil (82) des Sockels (81) befestigt ist und der Abstand zwischen dem Nachweismittel (10) und der Oberfläche (60) einstellbar ist.

2. Nachweissystem nach Anspruch 1, wobei das mindestens eine Nachweismittel (12, 13, 14, 15) mindestens einen ersten und einen zweiten Fotodetektor umfasst, die an einem ersten bzw. zweiten Ort positioniert sind, um im Bereich der lichtdurchlässigen Zone des Behälters (100, 200) einen ersten und einen zweiten Reaktionslichtstrahl zu erfassen und dieses um einen ersten und einen zweiten Wert eines Analyseparameters zu erhalten, der für eine eventuelle mikrobielle Anwesenheit im Inhalt (101, 201) des Behälters (100, 200) repräsentativ ist.

3. Nachweissystem nach Anspruch 1, wobei das mindestens eine Nachweismittel (12, 13, 14, 15) einen ersten Fotodetektor (12), der dazu eingerichtet ist, ein erstes Reaktionslichtsignal zu erfassen, um einen Wert n eines ersten Analyseparameters zu erhalten, der für eine eventuelle mikrobielle Anwesenheit im Inhalt des Behälters (100, 200) repräsentativ ist, und einen zweiten Fotodetektor (13) umfasst, der dazu eingerichtet ist, einen zweiten Reaktionslichtstrahl zu erfassen, der vom ersten Reaktionslichtstrahl verschieden ist, um einen Wert m eines zweiten Analyseparameters zu erhalten, der für eine eventuelle mikrobielle Anwesenheit im Inhalt (101, 201) des Behälters (100, 200) repräsentativ ist.

4. Nachweissystem nach Anspruch 2 oder 3, wobei der erste Fotodetektor (12) eine rot gefilterte Fotodiode umfasst und der zweite Fotodetektor (13) eine grün gefilterte Fotodiode umfasst.

5. Nachweissystem (1) nach den Ansprüchen 1 bis 4, wobei die Steuervorrichtung (50) umfasst:
a) ein Speichermittel zum Speichern eines ersten Wertes des ersten und des zweiten Analyseparameters, der mit dem mindestens einen Nachweismittel (12, 13, 14, 15) erhalten wird, und eines zweiten Wertes des ersten und des zweiten Analyseparameters, der nach einer Zeitdauer erhalten wird und mit dem mindestens einen Nachweismittel (12, 13, 14, 15) bestimmt wird,
b) ein Vergleichsmittel zum Vergleichen des ersten und des zweiten Wertes des ersten und des zweiten Analyseparameters zum Ermitteln eines eventuellen mikrobiellen Wachstums im Inhalt des Behälters (100, 200) .

6. Nachweissystem (1) nach einem der Ansprüche 1 bis 5, wobei das Steuermittel (50) dazu eingerichtet ist, kontinuierlich Werte zu empfangen und zu speichern, die von dem mindestens einen Nachweismittel (12, 13, 14, 15) erhalten werden.

7. Nachweissystem (1) nach einem der Ansprüche 1 bis 6, wobei das Steuermittel (50) einen Alarm umfasst, um eine eventuelle mikrobielle Anwesenheit im Inhalt (101, 201) des Behälters (100, 200) anzugeben.

8. Verfahren zum Nachweis der Anwesenheit eines Mikroorganismus in einer Probe, die den mindestens einen Mikroorganismus enthalten könnte, wobei das Verfahren die folgenden Schritte umfasst:
a) Einlegen der Probe in einen Behälter (100, 200), der eine Wand mit mindestens einer lichtdurchlässigen Zone umfasst, wobei die Probe vor dem Einlegen oder nach dem Einlegen der Probe in den Behälter (100, 200) in Kontakt mit einem Kulturmilieu gebracht wird, wobei das mindestens eine Kulturmilieu dazu eingerichtet ist, das Wachstum des mindestens einen Mikroorganismus zu ermöglichen, wobei die Mischung der Probe und des Kulturmilieus den gesamten oder einen Teil des Inhalts (101, 201) des Behälters (100, 200) bilden,
b) eventuelles Bebrüten des Inhalts (100, 200) mit einer Temperatur und während einer Zeitspanne, die ausreichend sind, um das Wachstum des mindestens einen Mikroorganismus zu ermöglichen,
c) Messen mindestens eines Wertes n mindestens eines Analyseparameters, der für eine eventuelle mikrobielle Anwesenheit im Innern des Behälters (100, 200) repräsentativ ist, mithilfe eines Nachweissystems (1) nach einem der Ansprüche 1 bis 7,
wobei der Schritt c) die Teilschritte umfasst, die bestehen in:
i. Beleuchten des Inhalts (101, 201) des Behälters (100, 200) mit der Lichtquelle (11) durch die lichtdurchlässige Zone und
ii. Erfassen des Reaktionslichtstrahls, der als Antwort auf die Beleuchtung des Inhalts (101, 201) ausgesendet wird, mit dem mindestens einen Nachweisemittel (12, 13, 14, 15), um den Wert n des Analyseparameters zu erhalten,
d) Vergleichen des Wertes n mit einem Schwellenwert ns desselben Analyseparameters, wobei der Schwellenwert ns bezeichnend für die Anwesenheit des mindestens einen Mikroorganismus in der Probe ist,
e) Ableiten der Anwesenheit oder Abwesenheit des mindestens einen Mikroorganismus in der Probe auf Grundlage des Vergleichsergebnisses.

9. Verfahren nach Anspruch 8, wobei:
- der Schritt c) Messen mindestens eines Analyseparameters umfasst, dessen Wert n zunimmt, wenn die Menge des Mikroorganismus zunimmt, wie beispielsweise Trübung,
- der Schritt d) Vergleichen des Wertes n mit einem Schwellenwert ns desselben Analyseparameters umfasst, wobei der Schwellenwert ns bezeichnend für die Anwesenheit des mindestens einen Mikroorganismus in der Probe ist,
- der Schritt e) Ableiten der Kontamination der Probe durch den mindestens einen Mikroorganismus umfasst, wenn der Wert n größer oder gleich dem Schwellenwert ns ist

10. Verfahren nach Anspruch 8, wobei:
- der Schritt c) Messen mindestens eines Analyseparameters umfasst, dessen Wert n abnimmt, wenn die Menge des Mikroorganismus abnimmt, wie beispielsweise Fluoreszenz,
- der Schritt d) Vergleichen des Wertes n mit einem Schwellenwert ns desselben Analyseparameters umfasst,
- der Schritt e) Ableiten der Kontamination der Probe durch den mindestens einen Mikroorganismus umfasst, wenn der Wert n kleiner oder gleich dem Schwellenwert ns ist.

11. Verfahren nach Anspruch 8, wobei:
- der Schritt c) Messen mindestens eines Analyseparameters, dessen einer Wert ni zunimmt, wenn die Menge des Mikroorganismus zunimmt, wie beispielsweise Trübung, und Messen mindestens eines zweiten Analyseparameters umfasst, dessen einer Wert n2 abnimmt, wenn die Menge des Mikroorganismus abnimmt, wie beispielsweise Fluoreszenz,
- der Schritt d) Vergleichen des Wertes ni mit einem dem ersten Analyseparameter zugehörigen Schwellenwert nsl und Vergleichen des Wertes n2 mit einem dem zweiten Analyseparameter zugehörigen Schwellenwert ns2 umfasst,
- der Schritt e) Ableiten der Kontamination der Probe durch den mindestens einen Mikroorganismus umfasst, wenn der Wert ni größer oder gleich dem Schwellenwert nsl ist und der Wert n2 kleiner oder gleich dem Schwellenwert ns2 ist.

## Claims

1. Detection system (1) comprising a device (10) for detecting the presence of at least one microorganism in the contents (101, 201) of a container (100, 200) comprising a wall with a translucent region, said detection device (10) comprising:
a) at least one light source (11), such as a light-emitting diode (LED), capable of illuminating the contents (101, 201) of the container (100, 200) by emitting an excitation light beam through the translucent region of the container (100, 200);
b) at least one detection means (12, 13, 14, 15), such as a photodiode, for detecting at least one reaction light beam emitted in response to the illumination of the contents (101, 201) of the container (100, 200); said at least one detection means (12, 13, 14, 15) being positioned at an angle of a set value in relation to the direction of the excitation light beam, to detect the reaction light beam;
said at least one light source (11) and said at least one detection means (12, 13, 14, 15) being equipped with at least one connection means (105, 205) located entirely outside the container (100, 200) to connect said at least one light source (11) and said at least one detection means (12, 13, 14, 15) to the wall of the container (100, 200), in the translucent region, said connection means (105, 205) making it possible to adapt the position of the detection device (10) on the wall of the container (100, 200),
c) a control device (50) connected to said at least one light source (11) and to said at least one detection means (12, 13, 14, 15) to control the excitation light beam emitted by said at least one light source (11) and/or to process and/or analyse said at least one reaction light beam emitted in response to the illumination of the contents of the container (100, 200), and detected by said at least one detection means (12, 13, 14, 15), **characterized in that** the detection system comprises
d) a support device comprising an L-shaped base (81), comprising a first portion (82) and a second portion (83) having a surface (60), the detection device (10) being fastened to the first portion (82) of the base (81), the distance between the detection device (10) and the surface (60) being adjustable.

2. Detection system according to Claim 1, wherein said at least one detection means (12, 13, 14, 15) comprises at least a first and a second photodetector positioned at a first and a second site, respectively, to detect, in the translucent region of the container (100, 200), a first and a second reaction light beam, in order to obtain a first and second value of an analysis parameter representative of a potential microbial presence in the contents (101, 201) of the container (100, 200) .

3. Detection system according to Claim 1, wherein said at least one detection means (12, 13, 14, 15) comprises a first photodetector (12) suitable for detecting a first reaction light signal, in order to obtain a value n of a first analysis parameter representative of a possible microbial presence in the contents of the container (100, 200), and a second photodetector (13) suitable for detecting a second reaction light beam, different from the first reaction light beam, in order to obtain a value m of a second analysis parameter representative of a potential microbial presence in the contents (101, 201) of the container (100, 200) .

4. Detection system according to Claim 2 or 3, wherein the first photodetector (12) comprises a red-filtered photodiode and the second photodetector (13) comprises a green-filtered photodiode.

5. Detection system (1) according to Claims 1 to 4, wherein the control device (50) comprises:
a) a storage medium for storing a first value of the first and second analysis parameter obtained with said at least one detection means (12, 13, 14, 15) and a second value of the first and second analysis parameter obtained after a set time period with said at least one detection means (12, 13, 14, 15),
b) a comparison means for comparing the first and second values of the first and second analysis parameter to determine a potential microbial growth in the contents of the container (100, 200) .

6. Detection system (1) according to one of Claims 1 to 5, wherein the control device (50) is suitable for continuously receiving and storing values obtained with said at least one detection means (12, 13, 14, 15).

7. Detection system (1) according to one of Claims 1 to 6, wherein the control device (50) comprises an alarm, to indicate a potential microbial presence in the contents (101, 201) of the container (100, 200).

8. Method for detecting the presence of at least one microorganism in a sample capable of containing said at least one microorganism, said method comprising the following steps:
a) introducing the sample into a container (100, 200) comprising a wall with at least one translucent region, said sample being placed in contact with a culture medium prior to the introduction or after the introduction of said sample into the container (100, 200), said at least one culture medium being suitable for enabling the growth of said at least one microorganism, the mixture of the sample and said culture medium forming all or part of the contents (101, 201) of the container (100, 200),
b) potentially incubating the container (100, 200) at a temperature and for a time period sufficient to allow the growth of said at least one microorganism,
c) measuring, using a detection system (1) according to one of Claims 1 to 7, at least one value n of at least one analysis parameter representative of a potential microbial presence inside the container (100, 200),
step c) comprising the sub-steps consisting in:
i. illuminating the contents (101, 201) of the container (100, 200) through the translucent region with the light source (11), and
ii. detecting the reaction light beam emitted in response to illuminating the contents (101, 201) with said at least one detection means (12, 13, 14, 15), in order to obtain said value n of the analysis parameter,
d) comparing said value n with a threshold value ns of the same analysis parameter, said threshold value ns indicating the presence of at least one microorganism in the sample,
e) deducing the presence or absence of said at least one microorganism, within the sample, based on the result of the comparison.

9. Method according to Claim 8, wherein:
- step c) comprises measuring at least one analysis parameter, the value n of which increases as the quantity of said microorganism increases, such as for example turbidity,
- step d) comprises comparing said value n with a threshold value ns of the same analysis parameter, said threshold value ns indicating the presence of at least one microorganism in the sample,
- step e) comprises deducing the contamination of the sample by said at least one microorganism if the value n is equal to or greater than the threshold value ns.

10. Method according to Claim 8, wherein:
- step c) comprises measuring at least one analysis parameter, the value n of which decreases as the quantity of said microorganism decreases, such as for example fluorescence,
- step d) comprises comparing said value n with a threshold value ns of the same analysis parameter,
- step e) comprises deducing the contamination of the sample by said at least one microorganism if the value n is smaller than or equal to the threshold value ns.

11. Method according to Claim 8, wherein:
- step c) comprises measuring at least a first analysis parameter, a value ni of which increases as the quantity of said microorganism increases, such as for example turbidity, and measuring at least a second analysis parameter, a value n2 of which decreases as the quantity of said microorganism decreases, such as for example fluorescence,
- step d) comprises comparing said value ni with a threshold value ns1, associated with the first analysis parameter, and comparing said value n2 with a threshold value ns2 associated with the second analysis parameter,
- step e) comprises deducing the contamination of the sample by said at least one microorganism if the value ni is equal to or greater than the threshold value ns1 and if the value n2 is smaller than or equal to the threshold value ns2.
